# EUROPEAN PATENT APPLICATION

(11) **EP 2 383 286 A1**
(43) Date of publication of application: **02.11.2011**
(21) Application number: 10305464.9
(22) Date of filing: 30.04.2010
(51) Int. Cl.: C07K 14/215, C07K 14/47, A61K 38/16, A61K 38/17

(54) **Methods and compositions for treatment of retinal degenerative diseases**

(71) Applicant: INSERM (Institut National de la Santé et de la Recherche Médicale), 75013 Paris (FR)
(72) Inventor: The designation of the inventor has not yet been filed
(74) Representative: Hirsch, Denise Marie

(57) **Abstract**

The present invention relates to an isolated nucleic acid molecule comprising i) a nucleotide sequence coding for a hyperpolarizing light-gated ion channel or pump gene from an archeon or for a light-active fragment of said gene, or the nucleotide sequence and ii) a nucleotide sequence coding for a RdCVF polypeptide for use in the treatment of a retinal degenerative disease.

## Description

### FIELD OF THE INVENTION:

The present invention relates to methods and compositions for treatment of retinal degenerative diseases.

### BACKGROUND OF THE INVENTION:

Photoreceptors are a specialized subset of retinal neurons that are responsible for vision. Photoreceptors consist of rods and cones which are the photosensitive cells of the retina. Each rod and cone elaborates a specialized cilium, referred to as an outer segment that houses the phototransduction machinery. The rods contain a specific light-absorbing visual pigment, rhodopsin. There are three classes of cones in humans, characterized by the expression of distinct visual pigments: the blue cone, green cone and red cone pigments. Each type of visual pigment protein is tuned to absorb light maximally at different wavelengths. The rod rhodopsin mediates scotopic vision (in dim light), whereas the cone pigments are responsible for photopic vision (in bright light). The red, blue and green pigments also form the basis of color vision in humans. The visual pigments in rods and cones respond to light and hyperpolarize photoreceptors. This visual information in then communicated to different bipolar neurons, which are then relayed by retinal ganglion neurons to produce a visual stimulus in the visual cortex.

In humans, a number of diseases of the retina involve the progressive degeneration and eventual death of photoreceptors, leading inexorably to blindness. Degeneration of photoreceptors, such as by inherited retinal dystrophies (e. g., retinal degenerative diseases), age related macular degeneration and other maculopathies, or retinal detachment, are all characterized by the progressive atrophy and loss of function of photoreceptor outer segments.

For instance, *Retinitis pigmentosa* refers to a diverse group of hereditary diseases which lead to retinal degeneration and incurable blindness. The disease is the result of mutations in genes expressed in rod photoreceptors; these then degenerate, causing loss of night vision. Subsequently, cone photoreceptors, which are responsible for colour and high acuity daytime vision, lose their photoreceptive outer segments, resulting in overall blindness. During this loss of sensitivity, many cones also degenerate but a significant number of cone cell bodies remains present in both humans and animals but it is not known whether these dormant cells can be reactivated or if information from them can still flow to downstream visual circuits.

Several treatments of retinal degenerative diseases have been investigated and include retinal transplants, artificial retinal implants, gene therapy, stem cells, nutritional supplements, and/or drug therapies. However, those strategies have shown limited success for restoring vision in patients affected with retinal degenerative diseases and/or can only apply to a very limited number of patients.

### SUMMARY OF THE INVENTION:

The present invention relates to an isolated nucleic acid molecule comprising i) a nucleotide sequence coding for a hyperpolarizing light-gated ion channel or pump gene from an archeon or for a light-active fragment of said gene, or the nucleotide sequence and ii) a nucleotide sequence coding for a RdCVF polypeptide for use in the treatment of a retinal degenerative disease.

### DETAILED DESCRIPTION OF THE INVENTION:

The present invention relates to an isolated nucleic acid comprising i) a nucleotide sequence coding for an archaebacterial halorhodopsin and ii) a nucleotide sequence coding for a Rod-derived Cone Viability Factor (RdCVF) polypeptide for use in the treatment of a retinal degenerative disease.

It has been demonstrated that using adeno-associated virus mediated gene delivery, specific expression of archaebacterial halorhodopsin in photoreceptors confers light sensitivity on dormant cones in mouse models of fast and slow forms of *Retinitis pigmentosa* (see e.g. International Patent Publication WO/2009/127705 and Jens Duebel, Volker Busskamp, David Balya, Mathias Seeliger, Peter Humphries, Martin Biel, Karl Deisseroth, Mathias Fradot, Serge Picaud, Botond Roska Expression of halorhodopsin in photoreceptors restores ON and OFF visual channels in retinal degeneration European Retina Meeting 2009). In another hand, it has been demonstrated that trophic factors that are capable of rescuing photoreceptors from cell death and/or restoring the function of dysfunctional (atrophic or dystrophic) photoreceptors represent useful therapies for the treatment of such conditions. For example, document WO02081513 has described the use of the Rod-derived Cone Viability Factor (RdCVF) for the treatment of retinal degenerative diseases. Accordingly, without whishing to be bound by any particular theory, the inventors believe that combination gene therapy based on a nucleotide sequence coding for a hyperpolarizing light-gated ion channel or pump gene from an archeon and a nucleotide sequence coding for a Rod-derived Cone Viability Factor (RdCVF) polypeptide is suitable for the treatment of retinal degenerative disease.

The term "retinal degenerative diseases" encompasses all diseases associated with photoreceptors degeneration. Retinal degenerative disease include but are not limited to Retinitis Pigmentosa, age-related macular degeneration, Bardet-Biedel syndrome, Bassen-Kornzweig syndrome, Best disease, choroidema, gyrate atrophy, Leber congenital amaurosis, Refsun syndrome, Stargardt disease or Usher syndrome.

In the context of the invention, the term "treating" or "treatment", as used herein, means reversing, alleviating, inhibiting the progress of, or preventing the disorder or condition to which such term applies, or one or more symptoms of such disorder or condition (e.g., retinal degenerative diseases).

According to the invention, the term "patient" or "patient in need thereof", is intended for a human or non-human mammal affected or likely to be affected with a retinal degenerative disease.

As intended herein the expression "isolated nucleic acid" refers to any type of isolated nucleic acid, it can notably be natural or synthetic, DNA or RNA, single or double stranded. In particular, where the nucleic acid is synthetic, it can comprise non-natural modifications of the bases or bonds, in particular for increasing the resistance to degradation of the nucleic acid. Where the nucleic acid is RNA, the modifications notably encompass capping its ends or modifying the 2' position of the ribose backbone so as to decrease the reactivity of the hydroxyl moiety, for instance by suppressing the hydroxyl moiety (to yield a 2'-deoxyribose or a 2'-deoxyribose-2'-fluororibose), or substituting the hydroxyl moiety with an alkyl group, such as a methyl group (to yield a 2'-O-methyl-ribose).

The term "archaebacterial halorhodopsin" or "NpHR" refers to a light-driven ion pump, specific for chloride ions, and found in phylogenetically ancient archaea, known as halobacteria. It is a seven-transmembrane protein of the retinylidene protein family, homologous to the light-driven proton pump bacteriorhodopsin, and similar in tertiary structure (but not primary sequence structure) to vertebrate rhodopsins, the pigments that sense light in the retina. Examples of archaebacterial halorhodopsin include but are not limited to *Natronomonas pharaonis* halorhodopsin and enhanced *Natronomonas pharaonis* halorhodopsin that are described in Gradinaru, V., Thompson, K. R. & Deisseroth, K. eNpHR: a Natronomonas halorhodopsin enhanced for optogenetic applications. Brain Cell Biol 36, 129-39 (2008). Other examples include those described in the International Patent Publication n°WO/2009/127705. Term also include polypeptides that are homologous to archaebacterial halorhodopsin.

Two amino acid sequences or nucleic acid sequences are "substantially homologous" or "substantially similar" when greater than 80 %, preferably greater than 85 %, preferably greater than 90 % of the amino acids or nucleic acid sequences are identical, or greater than about 90 %, preferably grater than 95 %, are similar (functionally identical). To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences. In one embodiment, the two sequences are the same length. The determination of percent identity between two sequences can be accomplished using a mathematical algorithm. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

The term "Rod-derived Cone Viability Factor (RdCVF) polypeptide" refers to any polypeptide that is encoded by Rod-derived Cone Viability Factor genes family. Said family include the RdCVF gene, also called thioredoxin-like 6 (Txnl6) or Nucleoredoxin like (Nxnl1) or any gene that is paralogous to RdCVF. Therefore the term encompasses polypeptides that are encoded by RdCVF gene such as described in the international Patent Application WO2081513, including the two distinct splice variants corresponding to RdCVF-L (long) and RdCVF-S (short). The term also encompasses the polypeptides encoded by RdCVF2 gene that is paralogous to RdCVF, such as described in the International Patent Application W02008/1148860, including the two distinct splice variants corresponding to RdCVF2-L (long) and RdCVF2-S (short). RdCVF and RdCVF2 sequences and gene structures are highly similar between both. The term also includes polypeptides that are homologous to the polypeptides (RDCVF1 or 2) as above described.

The nucleic acid according to the invention can be amplified using cDNA, mRNA or genomic DNA as a template and appropriate oligonucleotide primers according to standard The nucleic acid so amplified can be cloned into an appropriate vector and characterized by DNA sequence analysis. Furthermore, nucleic acids of the invention can be prepared by standard synthetic techniques, e.g., using an automated DNA synthesizer.

Nucleic acids of the invention may be delivered to the invention alone or in association with a vector.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked.

In general, the vectors useful in the invention include, but are not limited to, plasmids, phagemids, viruses, other vehicles derived from viral or bacterial sources that have been manipulated by the insertion or incorporation of nucleic acid according to the invention. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Murry, "Methods in Molecular Biology," vol.7, Humana Press, Inc., Cliffton, N.J., 1991..

Preferred viruses according to the invention are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms (Wu Z, Asokan A, Samulski RJ: Adeno-associated virus serotypes: vector toolkit for human gene therapy. Mol Ther 14:316-327, 2006). Recombinant AAV are derived from the dependent parvovirus AAV2 (Choi VW, Samulski RJ, McCarty DM: Effects of adeno-associated virus DNA hairpin structure on recombination. J Virol 79:6801-6807, 2005). The adeno-associated virus type 1 to 12 can be engineered to be replication deficient and is capable of infecting a wide range of cell types and species (Wu Z, Asokan A, Samulski RJ: Adeno-associated virus serotypes: vector toolkit for human gene therapy. Mol Ther 14:316-327, 2006). It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion and most recombinant adenovirus are extrachromosomal. In the sheltered environment of the retina, AAV vectors are able to maintain high levels of transgene expression in the retinal pigmented epithelium (RPE), photoreceptors, or ganglion cells for long periods of time after a single treatment. Each cell type can be specifically targeted by choosing the appropriate combination of AAV serotype, promoter, and intraocular injection site (Dinculescu et al., Hum Gene Ther. 2005 Jun;16(6):649-63 and Lebherz, C., Maguire, A., Tang, W., Bennett, J. & Wilson, J. M. Novel AAV serotypes for improved ocular gene transfer. J Gene Med 10, 375-82 (2008)). In a preferred embodiment, AAV serotype 8 is particularly suitable.

Non-viral administration of nucleic acid in vivo has been accomplished by a variety of methods These include lipofectin/liposome fusion Proc Natl Acad Sci 84, pp 7413-7417 (1993), polylysine condensation with and without adenovirus enhancement Human Gene Therapy 3, pp 147-154 (1992), and transferrin transferring receptor delivery of nucleic acid to cells Proc Natl Acad Sci 87, pp 3410-3414 (1990) The use of a specific composition consisting of polyacrylic acid has been disclosed in WO 94/24983 Naked DNA has been administered as disclosed in W090/1 1092.

In certain embodiments, the use of liposomes and/or nanoparticles is contemplated for the introduction of the donor nucleic acid targeting system into host cells.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm Sonication of MLVs results in the formation of small unilamellar vesicles (SUVs) with diameters in the range of 200 to 500 Å, containing an aqueous solution in the core.

Synthetic cationic lipids designed to limit the difficulties and dangers encountered with liposome mediated transfection can be used to prepare liposomes for in vivo transfection of a gene encoding a marker. The use of cationic lipids may promote encapsulation of negatively charged nucleic acids, and also promote fusion with negatively charged cell membranes (Felgner et al., 1989).

Alternatively, one of the simplest and the safest way to deliver the nucleic acid according to the invention across cell membranes in vivo may involve the direct application of high concentration free or naked polynucleotides (typically mRNA or DNA). By "naked DNA (or RNA)" is meant a DNA (RNA) molecule which has not been previously complexed with other chemical moieties. Naked DNA uptake by animal cells may be increased by administering the cells simultaneously with excipients and the nucleic acid. Such excipients are reagents that enhance or increase penetration of the DNA across cellular membranes and thus delivery to the cells delivery of the therapeutic agent. Various excipients have been described in the art, such as surfactants, e.g. a surfactant selected form the group consisting of Triton X-100, sodium dodecyl sulfate, Tween 20, and Tween 80; bacterial toxins, for instance streptolysin O, cholera toxin, and recombinant modified labile toxin of E coli; and polysaccharides, such as glucose, sucrose, fructose, or maltose, for instance, which act by disrupting the osmotic pressure in the vicinity of the cell membrane. Other methods have been described to enhance delivery of free polynucleotides, such as blocking of polynucleotide inactivation via endo- or exonucleolytic cleavage by both extra- and intracellular nucleases.

In a preferred embodiment, the nucleic acid according to the invention is under the control of a heterologous regulatory region, e.g., a heterologous promoter. The promoter can be, e.g., a photoreceptor specific promoter, such as the three versions of the human red cone opsin promoter (PR0.5, 3LCR-PR0.5 and PR2.1), the human blue cone opsin promoter HB569 (Gene Ther. 2008 Jul;15(14):1049-55. Epub 2008 Mar 13., Targeting gene expression to cones with human cone opsin promoters in recombinant AAV. Komáromy AM, Alexander JJ, Cooper AE, Chiodo VA, Glushakova LG, Acland GM, Hauswirth WW, Aguirre GD); three photoreceptor specifc promoters (interphotoreceptor retinoid binding protein-IRPB 1783; guanylate cyclase activating protein 1-GCAP292; rhodopsin-mOP500) '(Mol Vis. 2007 Oct 18;13:2001-11. Targeted expression of two proteins in neural retina using self-inactivating, insulated lentiviral vectors carrying two internal independent promoters. Semple-Rowland SL, Eccles KS, Humberstone EJ.) the human rhodopsin kinase (RK) promoter (Invest Ophthalmol Vis Sci. 2007 Sep;48(9):3954-61. AAV-mediated expression targeting of rod and cone photoreceptors with a human rhodopsin kinase promoter. Khani SC, Pawlyk BS, Bulgakov OV, Kasperek E, Young JE, Adamian M, Sun X, Smith AJ, Ali RR, Li T.) ; the promoter for the alpha subunit of cone transducin or the cone photoreceptor regulatory element 1 (CPRE-1) a novel 20-bp enhancer element in the TalphaC promoter (J Biol Chem. 2008 Apr 18;283(16):10881-91. Epub 2008 Feb 13. A novel, evolutionarily conserved enhancer of cone photoreceptor-specific expression. Smyth VA, Di Lorenzo D, Kennedy BN.), the promoter of the orphan nuclear receptor Nr2e3; the promoter of human retinal guanylate cyclase 1 (retGC1), and the cone transcription factor *Tr*β*2* [(Peng and Chen, 2005; Oh et al., 2007) promoter for the beta subunit of the phosphodiesterase, *PDE6B* (Mali et al., 2007). The promoter can also be selected form the group of genes consisting of human rhodopsin (hRHO), human red opsin (hRO), human green opsin and mouse cone arrestin-3 (mCAR). In a preferred embodiment, mouse cone arrestin-3 (mCAR) is particularly suitable.

Suitable methods, i.e., invasive and noninvasive methods, of administering a nucleic acid according to the invention so as to contact a photoreceptor are well known in the art. Although more than one route can be used to administer a nucleic acid according to the invention, a particular route can provide a more immediate and more effective reaction than another route. Accordingly, the described routes of administration are merely exemplary and are in no way limiting. Accordingly, the methods are not dependent on the mode of administering the nucleic acid of the invention to an animal, preferably a human, to achieve the desired effect. As such, any route of administration is appropriate so long as the nucleic acid of the invention contacts a photoreceptor. The nucleic acid of the invention can be appropriately formulated and administered in the form of an injection, eye lotion, ointment, implant and the like. The nucleic acid of the invention can be applied, for example, systemically, topically, subconjunctivally, intraocularly, retrobulbarly, periocularly, subretinally, or suprachoroidally. In certain cases, it may be appropriate to administer multiple applications and employ multiple routes, e.g., subretinal and intravitreous, to ensure sufficient exposure of photoreceptors to the nucleic acid of the invention. Multiple applications of the nucleic acid of the invention may also be required to achieve the desired effect.

Depending on the particular case, it may be desirable to non-invasively administer the nucleic acid according to the invention to a patient. For instance, if multiple surgeries have been performed, the patient displays low tolerance to anesthetic, or if other ocular-related disorders exist, topical administration of the nucleic acid according to the invention may be most appropriate. Topical formulations are well known to those of skill in the art. Such formulations are, suitable in the context of the present invention for application to the eye. The use of patches, corneal shields (see, e.g., U.S. Patent 5,185,152), and ophthalmic solutions (see, e.g., U.S. Patent 5,710,182) and ointments, e.g., eye drops, is also within the skill in the art. The nucleic acid according to the invention can also be administered non-invasively using a needleless injection device, such as the Biojector 2000 Needle-Free Injection Management System@ available from Bioject, Inc.

The nucleic acid according to the invention is preferably present in or on a device that allows controlled or sustained release of the nucleic acid according to the invention, such as an ocular sponge, meshwork, mechanical reservoir, or mechanical implant. Implants (see, e.g., U.S. Patents 5,443,505, 4,853,224 and 4,997,652), devices (see, e.g., U.S. Patents 5,554,187, 4,863,457, 5,098,443 and 5,725,493), such as an implantable device, e.g., a mechanical reservoir, an intraocular device or an extraocular device with an intraocular conduit, or an implant or a device comprised of a polymeric composition are particularly useful for ocular administration of the nucleic acid according to the invention. The nucleic acid according to the invention of the present inventive methods can also be administered in the form of sustained-release formulations (see, e.g., U.S.Patent 5,378,475) comprising, for example, gelatin, chondroitin sulfate, a polyphosphoester, such as bis-2-hydroxyethyl-terephthalate (BHET), or a polylacticglycolic acid.

Alternatively, the nucleic acid according to the invention can be administered using invasive procedures, such as, for instance, intravitreal injection or subretinal injection optionally preceded by a vitrectomy. Subretinal injections can be administered to different compartments of the eye, i.e., the anterior chamber. While intraocular injection is preferred, injectable compositions can also be administered intramuscularly, intravenously, and intraperitoneally. Pharmaceutically acceptable carriers for injectable compositions are well-known to those of ordinary skill in the art (see Pharmaceutics and Pharmacy Practice, J.B. Lippincott Co., Philadelphia, PA, Banker and Chalmers, eds., pages 238-250 (1982), and ASHP Handbook on Injectable Drugs, Toissel, 41h ed.,.pages 622-630 (1986)). The nucleic acid according to the invention can also be administered in vivo by particle bombardment, i.e., a gene gun. Preferably, the nucleic acid according to the invention is administered via an ophthalmologic instrument for delivery to a specific region of an eye. Use of a specialized ophthalmologic instrument ensures precise administration of the nucleic acid according to the invention while minimizing damage to adjacent ocular tissue. Delivery of the nucleic acid according to the invention to a specific region of the eye also limits exposure of unaffected cells to nucleic acid of the invention, thereby reducing the risk of side effects. A preferred ophthalmologic instrument is a combination of forceps and subretinal needle or sharp bent cannula. Alternatively, the nucleic acid according to the invention may be injected directly into the vitreous, aqueous humour, ciliary body tissue(s) or cells and/or extra-ocular muscles by electroporation or iontophoresis means.

The dose of nucleic acid according to the invention administered to an animal, particularly a human, in accordance with the present invention should be sufficient to effect the desired response in the animal over a reasonable time frame. One skilled in the art will recognize that dosage will depend upon a variety of factors, including the age, species, the pathology in question, and condition or disease state. Dosage also depends on the nucleic acid to be expressed, as well as the amount of ocular tissue about to be affected or actually affected by the retinal degenerative disease. The size of the dose also will be determined by the route, timing, and frequency of administration as well as the existence, nature, and extent of any adverse side effects that might accompany the administration of a particular nucleic acid according to the invention and the desired physiological effect. It will be appreciated by one of ordinary skilled in the art that various conditions or disease states, in particular, chronic conditions or disease states, may require prolonged treatment involving multiple administrations.

The nucleic acid of the invention is administered in a pharmaceutical composition, which comprises a pharmaceutically acceptable carrier and the nucleic acid(s) of the invention. Any suitable pharmaceutically acceptable carrier can be used within the context of the present invention, and such carriers are well known in the art. The choice of carrier will be determined, in part, by the particular site to which the composition is to be administered and the particular method used to administer the composition.

Suitable formulations include aqueous and non-aqueous solutions, isotonic sterile solutions, which can contain anti-oxidants, buffers, bacteriostats, and solutes that render the formulation isotonic with the blood or intraocular fluid of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampoules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets of the kind previously described. Preferably, the pharmaceutically acceptable carrier is a buffered saline solution. More preferably, the nucleic acid of the invention for use in the present inventive methods is administered in a pharmaceutical composition formulated to protect the nucleic acid of the invention from damage prior to administration. For example, the pharmaceutical composition can be formulated to reduce loss of the nucleic acid of the invention on devices used to prepare, store, or administer the nucleic acid of the invention, such as glassware, syringes, or needles. The pharmaceutical composition can be formulated to decrease the light sensitivity and/or temperature sensitivity of the nucleic acid of the invention. To this end, the pharmaceutical composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a pharmaceutical composition will extend the shelf life of the nucleic acid, facilitate administration, and increase the efficiency of the methods of the invention. In this regard, a pharmaceutical composition also can be formulated to enhance transduction efficiency.

In addition, one of ordinary skill in the art will appreciate that the nucleic acid can be present in a composition with other therapeutic or biologically-active agents. For example, therapeutic factors useful in the treatment of a particular indication can be present. For instance, if treating vision loss, hyaluronidase can be added to a composition to effect the break down of blood and blood proteins in the vitreous of the eye. Factors that control inflammation, such as ibuprofen or steroids, can be part of the composition to reduce swelling and inflammation associated with in vivo administration of the nucleic acid according to the invention and ocular distress. Immune system suppressors can be administered in combination to reduce any immune response to the nucleic acid itself. Similarly, vitamins and minerals, anti-oxidants, and micronutrients can be co-administered. Antibiotics, i.e., microbicides and fungicides, can be present to reduce the risk of infection associated with gene transfer procedures and other disorders.

The present invention also relates to pharmaceutical compositions comprising an isolated nucleic acid according to the invention.

The present invention also relates to a kit of parts comprising a first compound consisting of an isolated nucleic acid coding for an archaebacterial halorhodopsin and a second compound consisting of an isolated nucleic acid coding for a RdCVF polypeptide for use in the treatment of a retinal degenerative disease.

The present invention also relates to a kit of parts comprising a first compound consisting of a nucleic acid and coding for an archaebacterial halorhodopsin and a second compound consisting of RdCVF polypeptide.

The present invention also relates to a method for treating a retinal degenerative disease comprising administering a patient in need thereof with a therapeutically effective amount of an isolated nucleic acid according to the invention.

A "therapeutically effective amount" is intended for a minimal amount of active agent (e.g., a nucleic acid according to the invention) which is necessary to impart therapeutic benefit to a patient. For example, a "therapeutically effective amount" to a mammal is such an amount which induces, ameliorates or otherwise causes an improvement in the pathological symptoms, disease progression or physiological conditions associated with or resistance to succumbing to a disorder.

In a particular embodiment, the present invention relates to a method for treating a retinal degenerative disease comprising administering a patient in need thereof with a therapeutically effective amount of an isolated nucleic acid according to the invention

The present invention also relates to method for treating a retinal degenerative disease comprising administering a patient in need thereof with a therapeutically effective amount of an isolated nucleic acid coding for an archaebacterial halorhodopsin and a therapeutically effective amount of an isolated nucleic acid coding for a RdCVF polypeptide

In another particular embodiment, the present invention relates to a method for treating a retinal degenerative disease comprising administering a patient in need thereof with a therapeutically effective amount of an isolated nucleic acid coding for an archaebacterial halorhodopsin and with a therapeutically effective amount of a RdCVF polypeptide.

The present invention also relates to a combination of an isolated nucleic acid coding for an archaebacterial halorhodopsin and an isolated nucleic acid sequence coding for a polypeptide for use in the treatment of a retinal degenerative disease.

The present invention also relates to a combination of an isolated nucleic acid coding for an archaebacterial halorhodopsin and a RdCVF polypeptide for use in the treatment of a retinal degenerative disease.

The present invention also relates to pharmaceutical compositions comprising a first compound consisting of an isolated nucleic acid coding for an archaebacterial halorhodopsin and a second compound consisting of an isolated nucleic acid sequence coding for a polypeptide for use in the treatment of a retinal degenerative disease.

The present invention also relates to pharmaceutical compositions comprising a first compound consisting of isolated nucleic acid coding for an archaebacterial halorhodopsin and a second compound consisting of a RdCVF polypeptide for use in the treatment of a retinal degenerative disease.

### REFERENCES:

Throughout this application, various references describe the state of the art to which this invention pertains. The disclosures of these references are hereby incorporated by reference into the present disclosure.

## Claims

1. An isolated nucleic acid comprising i) a nucleotide sequence coding for an archaebacterial halorhodopsin and ii) a nucleotide sequence coding for a Rod-derived Cone Viability Factor (RdCVF) polypeptide for use in the treatment of a retinal degenerative disease.

2. The isolated nucleic acid according to claim 1 which is delivered in association with a vector.

3. The isolated nucleic acid according to claim 2 wherein said vector is a viral vector selected from the group consisting of moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus, adenoviruses and adeno-associated (AAV) viruses.

4. The isolated nucleic acid according to any of claims 1 to 3 which is under the control of a heterologous promoter such as photoreceptor specific promoter.

5. The isolated nucleic acid according to any of claims 1 to 4 wherein said retinal degenerative disease is selected from the group consisting of Retinitis Pigmentosa, age-related macular degeneration, Bardet-Biedel syndrome, Bassen-Kornzweig syndrome, Best disease, choroidema, gyrate atrophy, Leber congenital amaurosis, Refsun syndrome, Stargardt disease or Usher syndrome.

6. A pharmaceutical composition comprising an isolated nucleic acid according to any of claims 1 to 5.

7. A kit of parts comprising a first compound consisting of an isolated nucleic acid coding for an archaebacterial halorhodopsin and a second compound consisting of an isolated nucleic acid coding for a RdCVF polypeptide for use in the treatment of a retinal degenerative disease.

8. A kit of parts comprising a first compound consisting of a nucleic acid and coding for an archaebacterial halorhodopsin and a second compound consisting of RdCVF polypeptide.

9. A combination of an isolated nucleic acid coding for an archaebacterial halorhodopsin and an isolated nucleic acid sequence coding for a polypeptide for use in the treatment of a retinal degenerative disease.

10. A combination of an isolated nucleic acid coding for an archaebacterial halorhodopsin and a RdCVF polypeptide for use in the treatment of a retinal degenerative disease.

11. A pharmaceutical composition comprising a first compound consisting of an isolated nucleic acid coding for an archaebacterial halorhodopsin and a second compound consisting of an isolated nucleic acid sequence coding for a polypeptide for use in the treatment of a retinal degenerative disease.

12. A pharmaceutical composition comprising a first compound consisting of isolated nucleic acid coding for an archaebacterial halorhodopsin and a second compound consisting of a RdCVF polypeptide for use in the treatment of a retinal degenerative disease.
